# EUROPÄISCHE PATENTANMELDUNG

(11) **EP 3 878 485 A1**
(43) Veröffentlichungstag der Anmeldung: **15.09.2021**
(21) Anmeldenummer: 21167671.3
(22) Anmeldetag: 23.04.2015
(51) Int. Cl.: A61L 31/18, A61L 31/10, A61L 31/12, A61M 25/09

(54) **FÜHRUNGSDRAHT MIT EINEM KÖRPER AUS VON EINEM SCHRUMPFSCHLAUCH UMHÜLLTEN KOMPOSITMATERIAL**

(30) Priorität: 23.04.2014 DE 102014005777; 28.04.2014 DE 102014005994
(62) Teilanmeldung aus: 15735845.8
(71) Anmelder: MaRVis Interventional GmbH, 82494 Krün (DE)
(72) Erfinder: Düring, Klaus, 82494 Krün (DE); Dlaikan-Campos, Nasib, 52146 Würselen (DE); Van Dijk, Maarten, 5171 Kaatsheuvel (NL)
(74) Vertreter: Schüssler, Andrea

(57) **Zusammenfassung**

Ein erfindungsgemäßer stabförmiger Körper umfasst einen Zentralabschnitt und einen Peripherabschnitt, wobei der Zentralabschnitt im Zentrum des stabförmigen Körpers angeordnet ist und vom Peripherabschnitt umschlossen ist. Sowohl der Zentralabschnitt als auch der Peripherabschnitt erstrecken sich über im Wesentlichen die gesamte Länge des stabförmigen Körpers. Der Zentralabschnitt weist zumindest ein in ein nicht-ferromagnetisches Matrixmaterial eingebettetes, nicht-metallisches Faserbündel auf. Das Matrixmaterial ist mit Markerpartikeln dotiert. Der Peripherabschnitt weist zumindest ein in ein undotiertes, nicht-ferromagnetisches Matrixmaterial auf. Der Durchmesser des Zentralabschnitts ist kleiner oder gleich 0,2 mm, bevorzugt kleiner oder gleich 0,15 mm und noch mehr bevorzugt kleiner oder gleich 0,1 mm und insbesondere kleiner oder gleich 0,08 mm.

## Beschreibung

Die vorliegende Erfindung betrifft einen stabförmigen Körper und ein medizinisches Instrument. Insbesondere betrifft die vorliegende Erfindung ein medizinisches Instrument, das zumindest einen stabförmigen Körper umfasst, wobei der stabförmige Körper bzw. das medizinische Instrument mittels Magnetresonanztomographie detektierbar ist.

Aus der WO 2007/000148 A2 geht ein stabförmiger Körper hervor, der zur Ausbildung medizinischer Instrumente, wie zum Beispiel Katheter oder Führungsdrähte für Katheter, vorgesehen ist. Dieser stabförmige Körper besteht aus einem oder mehreren Filamenten und einem nicht-ferromagnetischen Matrixwerkstoff, wobei der Matrixwerkstoff die Filamente umschließt. In den Matrixwerkstoff ist eine Dotierung aus magnetresonanztomographische Artefakte erzeugenden Partikeln eingebracht.

In der WO 2009/141165 A2 ist ein medizinisches Instrument offenbart, das in einen menschlichen oder tierischen Körper einführbar ist, wobei das medizinische Instrument einen Instrumentenkörper aufweist. Der Instrumentenkörper weist zumindest einen schlecht elektrisch leitenden stabförmigen Körper auf, der aus einem Matrixwerkstoff und nicht-metallischen Filamenten ausgebildet ist. Dieses medizinische Instrument zeichnet sich dadurch aus, dass der stabförmige Körper mit einem Röntgenmarker dotiert ist, und das medizinische Instrument einen MR-Marker aufweist.

Aus der WO 2012/052159 A2 geht ein stabförmiger Körper und ein medizinisches Instrument hervor. Der stabförmige Körper umfasst ein oder mehrere nicht-metallische Filamente und ein nicht-ferromagnetisches Matrixmaterial. Das Matrixmaterial umschließt und/oder verklebt die Filamente. Markerpartikel zum Erzeugen eines Signals in der Magnetresonanz- oder in der Röntgenbildgebung sind in den stabförmigen Körper eingebracht.

Aus der WO 2013/041235 A1 geht eine medizinische Vorrichtung hervor, die während des Einbringens bzw. während der Positionierung im menschlichen und/oder tierischen Körper mittels MRT sichtbar gemacht werden kann und deren Biegesteifigkeit veränderbar ist. Die medizinische Vorrichtung weist eine langgestreckte, rohrförmige Lead-Sonde und eine darin anordbare langgestreckte Seele auf, die aus nicht-metallischen Filamenten und einem Matrixwerkstoff ausgebildet ist.

Eine ausführliche Erläuterung der Magnetresonanztomographie (MRT) ist im Internet unter http:/en.wikipedia.org/wiki/MRT erhältlich.

Derartige stabförmige Körper sind dahingehend vorteilhaft, dass durch Einbetten eines oder mehrerer stabförmiger Körper in ein Hüllmaterial ein medizinisches Instrument ausbildbar ist oder die stabförmigen Körper als Seele für Lead-Sonden in medizinischen Instrumenten eingesetzt werden können. Die stabförmigen Körper können unterschiedliche Dotierungsmaterialien enthalten. Durch Verwendung unterschiedlicher Arten von stabförmigen Körpern sind medizinische Vorrichtungen mit unterschiedlichen Dotierungsmaterialien (= Marker) herstellbar. Dadurch haben diese unterschiedliche Eigenschaften bezüglich ihrer Sichtbarkeit in der Röntgen- oder Magnetresonanzbildgebung. Diese verschiedenen medizinischen Instrumente können durch die gleichen Verfahren im gleichen Produktionsablauf hergestellt werden, wobei lediglich ein oder mehrere stabförmige Körper ausgetauscht werden müssen. Dadurch ist es möglich, unterschiedliche Arten von medizinischen Instrumenten wirtschaftlich herzustellen, selbst wenn diese lediglich in geringen Stückzahlen erzeugt werden.

Eine Aufgabe der vorliegenden Erfindung ist es, derartige stabförmige Körper weiter zu verbessern.

Eine weitere Aufgabe ist es, stabförmige Körper bereitzustellen, die sich leichter und in verbesserter Qualität herstellen lassen.

Eine weitere Aufgabe ist es, stabförmige Körper mit verbesserten mechanischen Eigenschaften bereitzustellen.

Eine weitere Aufgabe ist es, durch eine verbesserte Zusammensetzung der stabförmigen Körper verbesserte medizinische Instrumente, insbesondere MRkompatible Führungsdrähte, bereitzustellen, die die Eigenschaften der verbesserten stabförmigen Körper optimal ausnutzen.

Eine weitere Aufgabe ist es, verbesserte stabförmige Körper und medizinische Instrumente bereitzustellen, die eine verbesserte Sichtbarmachung in der Magnetresonanztomographie ermöglichen und insbesondere ein schärferes, enger begrenztes MRT-Artefakt erzeugen.

Eine weitere Aufgabe ist es, verbesserte Mikro-MR-Führungsdrähte bereitzustellen.

Die Erfindung weist zur Lösung dieser Aufgaben die in den unabhängigen Patentansprüchen angegebenen Merkmale auf. Vorteilhafte Ausgestaltungen davon sind in den jeweiligen Unteransprüchen angegeben.

Gemäß einem ersten Aspekt umfasst der stabförmige Körper einen Zentralabschnitt und einen Peripherabschnitt, wobei der Zentralabschnitt im Zentrum des stabförmigen Körpers angeordnet ist und vom Peripherabschnitt umschlossen ist. Sowohl der Zentralabschnitt als auch der Peripherabschnitt erstrecken sich über im Wesentlichen die gesamte Länge des stabförmigen Körpers. Der Zentralabschnitt weist zumindest ein in ein nicht-ferromagnetisches Matrixmaterial eingebettetes, nicht-metallisches Faserbündel auf. Das Matrixmaterial ist mit Markerpartikeln dotiert. Der Peripherabschnitt weist ein undotiertes, nicht-ferromagnetisches Matrixmaterial auf. Der Durchmesser des Zentralabschnitts ist kleiner oder gleich 0,2 mm, bevorzugt kleiner oder gleich 0,15 mm und noch mehr bevorzugt kleiner oder gleich 0,1 mm und insbesondere kleiner oder gleich 0,08 mm.

Dadurch, dass lediglich das Matrixmaterial des sehr kleinen Zentralabschnitts mit MR-Markerpartikeln dotiert ist, wird bei der Magnetresonanztomographie ein besonders schmales und scharfes Artefakt erzeugt.

Eine derart konzentrierte Anordnung einer gegenüber dem Stand der Technik geringeren Menge von MR-Markerpartikeln ist vorteilhaft gegenüber derjenigen mit einer höheren Menge von MR-Markerpartikeln, die im gesamten stabförmigen Körper verteilt sind, da die Voxel entlang der konzentrierten Anordnung von MR-Partikeln geschwärzt werden, aber diese Voxel den gleichen Schwärzungsgrad aufweisen wie bei einer größeren Menge an MR-Markerpartikeln, die über einen entsprechend größeren Bereich verteilt ist. Dadurch werden in der Breite weniger Voxel geschwärzt und ein schmaleres Abbild des medizinischen Instruments erreicht, so dass weniger Zielgewebe durch Schwärzung im MRT-Bild überdeckt werden kann.

Daher ist es mit einem erfindungsgemäßen stabförmigen Körper mit MR-Marker-Dotierung nur im Zentralabschnitt möglich, in der Magnetresonanztomographie ein annähernd ebenso schmales Abbild zu erzeugen wie mit einem metallischen Führungsdraht in einem Röntgenbildgebungsverfahren. Dies gilt insbesondere, wenn das medizinische Instrument lediglich einen einzigen dotierten stabförmigen Körper aufweist oder bei mehreren stabförmigen Körpern nur der zentral angeordnete stabförmige Körper MR-Marker aufweist. Vorzugsweise ist dann der mit zentrierter Anordnung von Markerpartikeln ausgebildete stabförmige Körper im Zentrum des Führungsdrahtes angeordnet. Hierdurch wird der Abstand zwischen dem dotierten Zentralabschnitt und der Oberfläche des Führungsdrahtes maximal gehalten. Dies führt dazu, dass Wasser- oder Fettmoleküle im zu untersuchenden Körper nicht näher als an die Oberfläche des Führungsdrahtes an den Zentralabschnitt heran gelangen können. Dadurch wird die Resonanz zwischen den MR-Markern und den Wassermolekülen gering gehalten, wodurch die von den MR-Markern erzeugten Artefakte klein sind und sich der Führungsdraht als schmaler Streifen in einem MR-Bildgebungsverfahren darstellt.

Zudem hat sich gezeigt, dass aufgrund der Konzentration der MR-Marker auf den Zentralabschnitt der Anteil der MR-Marker im Matrixmaterial über einen großen Bereich variieren kann, ohne dass dies nennenswert die Darstellung des Führungsdrahtes im MR-Bildgebungsverfahren beeinflusst. Bei Verwendung von Eisenpartikeln mit einer Korngröße von 0 bis 20 µm wurde bei einem Gewichtsverhältnis von ca. 1:5 bis ca. 1:30 zwischen Markern und Matrixmaterial im Wesentlichen die gleiche Darstellung erzielt. Es hat sich gezeigt, dass die lokale Konzentration auf einen an sich möglichst kleinen Bereich, d.h. den Zentralabschnitt wesentlich mehr Einfluss auf die Darstellung im MR-Bildgebungsverfahren hat als die Anteile an Markern im Matrixmaterial.

Der stabförmige Körper weist vorzugsweise einen Durchmesser von nicht mehr als 0,75 mm und insbesondere einen Durchmesser von 0,5 mm auf. Mit der Erfindung werden erstmals derartig dünne stabförmige Körper mit einer Struktur geschaffen, die einen Zentralabschnitt und einen Peripherabschnitt aufweisen.

Ein stabförmiger Körper ist ein einteiliger Vollmaterialkörper. Es ist kein rohr- oder schlauchförmiger Hohlkörper.

Vorzugsweise besteht der stabförmige Körper aus einem homogenen Matrixmaterial. Das Matrixmaterial des Peripherabschnitts und des Zentralabschnitts bestehen somit aus dem gleichen Materialtyp. Der bevorzugte Materialtyp ist Epoxidharz. Andere Materialtypen können aus anderen vorzugsweise auch chemisch reaktiven und funktionellen Polymeren ausgebildet sein. Geeignete Beispiele sind radikalische oder ionische Quervernetzungen von Polymeren, wie z.B. ungesättigten Polyestern, Veretherungen und Veresterungen von Polysachariden, Hydrolysen von Polyvinylestern oder Acetalisierungen von Polyvinylalkohol.

Vorzugsweise sind die nicht-metallischen Fasern im Peripherabschnitt etwa gleichmäßig bzgl. des Querschnitts angeordnet. Hierdurch wird auch bei einem dünnen oder kleinvolumigen Peripherabschnitt eine hohe Torsions- und Biegefestigkeit erzielt.

Es hat sich auch gezeigt, dass durch die Konzentration auf den Zentralabschnitt die gesamte Menge an MR-Marker sehr gering ist und trotzdem eine sehr gute Abbildung erzielt wird.

Auf diese Weise wird eine bestmögliche Darstellung eines medizinischen Instruments, insbesondere eines Führungsdrahtes, das einen derartigen stabförmigen Körper mit Dotierung nur im Zentralabschnitt umfasst, erzielt.

Zusätzlich zu den im Zentralabschnitt der stabförmigen Körper enthaltenen MR-Markerpartikeln kann auch ein separater MR-Spitzenmarker am distalen Endbereich der medizinischen Instrumente aufgebracht werden, der ein breiteres Artefakt, z.B. mit der zwei- bis dreifachen Breite des Artefakts über die gesamte Länge des medizinischen Instruments, im MRT-Bild erzeugt. Mit solch einem Spitzenmarker kann die Spitze des medizinischen Instruments und damit dessen distales Ende eindeutig im MRT-Bild identifiziert werden. Ist der Spitzenmarker nicht sichtbar im MRT-Bild, so befindet sich die Spitze nicht in der visualisierten Schicht der MRT-Aufnahme und die Schichteinstellung muss nachgestellt werden.

Der MR-Spitzenmarker wird bspw. durch Auftragen einer selbsthärtenden Polymerlösung, die MR-Markerpartikel enthält, hergestellt. Nach dem Aushärten bildet die Polymerlösung eine Schicht, die sich vorzugsweise über einen Längsbereich von einigen µm bis zu einigen mm erstreckt. Die Schicht kann alleine an einer Stirnfläche des stabförmigen Körpers aufgebracht sein, so dass die Längserstreckung der Schichtdicke entspricht. Die Schicht kann jedoch auch auf die Mantelfläche des stabförmigen Körpers aufgetragen sein, wobei dann die Längserstreckung vorzugsweise nicht länger als 5 mm und insbesondere nicht länger als 3 mm ist. Die Polymerlösung kann z.B. aus PEBAX oder einem Klebstoff bestehen. Als MR-Markerpartikel können alle hierin offenbarten MR-Marker verwendet werden, wobei Eisen-Markerpartikel bevorzugt werden. Der Spitzenmarker kann mit weiteren Schichten wie z.B. einer Hüllmaterialschicht überzogen werden. Bevorzugt ist eine solche weitere Überdeckung mit Polymermaterial.

Am distalen Ende der medizinischen Instrumente können auch mehrere Spitzenmarker aufgebracht werden, vorzugsweise in einem definierten Abstand voneinander. Auf diese Weise kann eine Messfunktion in das medizinische Instrument eingebaut werden, um die Länge z.B. einer Gefässstenose im Körper ausmessen zu können.

Die Größe und insbesondere die Breite des Spitzenmarker-Artefakts ist abhängig von der absoluten aufgetragenen Menge der MR-Markerpartikel und von der Schichtdicke der über dem Spitzenmarker befindlichen Material-/Polymerschicht, weil diese den Abstand zu den umgebenden Wasser- oder Fettmolekülen bestimmt.

Ein Faserbündel kann mindestens ein langgestrecktes oder mehrere langgestreckte Fasern und vorzugsweise mehrere langgestreckte Fasern umfassen, die parallel angeordnet oder miteinander verflochten oder verdrillt sind. Dadurch, dass das Faserbündel mindestens eine langgestreckte oder mehrere langgestreckte Fasern umfasst, verleiht das Faserbündel dem stabförmigen Körper eine hohe Festigkeit in longitudinaler Richtung. Durch eine derartige geordnete Ausbildung und Anordnung der Faserbündel im stabförmigen Körper wird eine bessere Produktqualität erreicht.

Das Faserbündel im Zentralabschnitt kann ein ht-Faserbündel sein und das Faserbündel im Peripherabschnitt kann ein Glasfaserbündel sein.

Ein ht-Faserbündel ist ein hochfestes Faserbündel. Typische Beispiele für ht-Faserbündel sind Aramidfasern und UHMWPE-Fasern (Ultra High Molecular Weight Polyethylen Fibers). ht-Faserbündel haben eine Zug- bzw. Reißfestigkeit von zumindest 20 cN/tex. Optional haben die ht-Faserbündel eine Zug- bzw. Reißfestigkeit von zumindest 32 cN/tex und insbesondere von zumindest 30 cN/tex.

Ein ht-Faserbündel ist hochflexibel bzw. biegsam und weist eine hohe Zug- bzw. Reißfestigkeit auf. Auf diese Weise ist sichergestellt, dass selbst wenn die stabförmigen Körper im menschlichen oder tierischen Körper während der medizinischen Intervention brechen, die gebrochenen Teile immer noch durch das ht-Faserbündel miteinander verbunden bleiben, wodurch das medizinische Instrument sicher entfernbar ist. Zudem verleiht das in das Matrixmaterial eingebettete ht-Faserbündel dem stabförmigen Körper eine gewisse Steifigkeit.

Glasfaserbündel sind steifer als ht-Faserbündel, so dass ein medizinisches Instrument, das sowohl ht-Faserbündel als auch Glasfaserbündel aufweist, bevorzugt ist.

Ein stabförmiger Körper, der sowohl ht-Faserbündel als auch Glasfaserbündel aufweist, kann hinsichtlich seiner Steifigkeit und Flexibilität und insbesondere bezüglich seiner Torsionssteifigkeit optimal eingestellt werden.

Die Anordnung zumindest eines Glasfaserbündels im Peripherabschnitt ermöglicht die höchstmögliche Steifigkeit. Das zumindest eine periphere Glasfaserbündel gibt dem stabförmigen Körper die notwendige Kompressions- und Biegesteifigkeit. Dadurch, dass zumindest ein ht-Faserbündel zentral auf einer neutralen Linie angeordnet ist, mindert das zumindest eine ht-Faserbündel die Kompressions- und die Biegesteifigkeit des stabförmigen Körpers nur minimal. Als neutrale Linie, auch Nulllinie genannt, bezeichnet man in der technischen Mechanik im Rahmen der Elastostatik die Schicht eines Querschnitts eines stabförmigen Körpers, deren Länge sich bei einem Biegevorgang nicht ändert. Dort verursacht die Biegung keine Zug- oder Druckspannungen. Die Fläche verläuft durch den geometrischen Schwerpunkt der Querschnittsfläche des stabförmigen Körpers.

Das zumindest eine Faserbündel im Zentralabschnitt kann auch ein Glasfaserbündel sein und das zumindest eine Faserbündel im Peripherabschnitt ein ht-Faserbündel. Diese Anordnung ist dann optimal, wenn der stabförmige Körper einen geringen Anteil an Glasfaser und einen hohen Anteil an ht-Fasern haben soll. In dieser Ausführungsform wird ein überwiegend aus der flexibleren ht-Faser bestehender stabförmiger Körper in der Kompressions- und Biegesteifigkeit durch die Glasfasern verstärkt.

Es ist vorteilhaft, die im größeren Anteil enthaltenen Fasern an der Oberfläche des stabförmigen Körpers und die im kleineren Anteil enthaltenen Fasern im Inneren anzuordnen, um eine homogene Oberfläche zu erhalten. Die Produktqualität wird dadurch wesentlich erhöht.

Die nicht-metallischen Faserbündel sind elektrisch nicht-leitende Fasern bzw. Filamente, so dass diese während der Magnetresonanztomographie verwendbar sind. Demgemäß schließt der Begriff "nicht-metallisches Faserbündel", wie er in der vorliegenden Beschreibung verwendet wird, jegliche elektrisch leitfähige Fasern, wie zum Beispiel dünne Metalldrähte oder ein Karbonfilament aus.

Vorzugsweise ist ein Faserbündel aus mehreren Fasern ausgebildet. Ein derartiges Faserbündel wird im Englischen als "roving" bezeichnet.

Wenn die Fasern des Faserbündels miteinander verdrillt sind, bilden diese ein Garn aus. Ein derartiges Faserbündel wird im Englischen als "yarn" bezeichnet.

Alle Faserbündel im stabförmigen Körper können ht-Faserbündel sein. Ein derart ausgebildeter stabförmiger Körper weist bestmögliche Eigenschaften hinsichtlich der Reißfestigkeit auf.

Weiterhin können alle Faserbündel im stabförmigen Körper Glasfaserbündel sein. Ein derartiger stabförmiger Körper weist bestmögliche Eigenschaften hinsichtlich der Kompressions- und Biegesteifigkeit auf.

Das nicht-ferromagnetische Matrixmaterial im Zentralabschnitt und im Peripherabschnitt kann das gleiche nicht-ferromagnetische Matrixmaterial sein. Als Matrixmaterial ist vorzugsweise Epoxidharz vorgesehen.

Die Markerpartikel im Zentralabschnitt sind vorzugsweise MR-Markerpartikel.

Im Zentralabschnitt können ein oder mehrere Faserbündel vorgesehen sein und im Peripherabschnitt können radial umlaufend um das Faserbündel des Zentralabschnitts und in etwa gleich beabstandet voneinander angeordnet zumindest drei bzw. vier bzw. fünf bzw. sechs bzw. sieben bzw. acht bzw. neun bzw. zehn bzw. elf bzw. zwölf Faserbündel vorgesehen sein.

Nach einem zweiten Aspekt der vorliegenden Erfindung umfasst ein medizinisches Instrument, bspw. ein Führungsdraht oder ein Katheter oder eine Seele für eine Lead-Sonde, insbesondere ein Mikro-Führungsdraht, einen stabförmigen Körper gemäß der vorstehenden Beschreibung.

Ein derartiger Mikro-Führungsdraht oder eine derartige Seele aus nur einem stabförmigen Körper erzielt eine maximale Biege- und Kompressionssteifigkeit, wobei durch das ht-Faserbündel die Reißfestigkeit sichergestellt wird.

Dadurch, dass lediglich das Matrixmaterial des Zentralabschnitts des stabförmigen Körpers des Führungsdrahtes mit MR-Markerpartikeln dotiert ist, wird bei der Magnetresonanztomographie ein besonders schmales und scharfes Artefakt erzeugt. Bzgl. der Vorteile des schmalen Artefakts wird auf die Erläuterungen zum erfindungsgemäßen stabförmigen Körper verwiesen.

Im Bereich eines als Spitze ausgebildeten distalen Endes eines Führungsdrahtes oder einer Seele kann der Anteil von Glasfasern des Faserbündels kleiner sein als im übrigen Führungsdraht.

Dadurch weist ein derartiger Mikro-Führungsdraht oder eine derartige Seele eine flexible Spitze am distalen Ende auf, die dadurch ausgebildet wird, dass dort der Anteil an Glasfasern geringer als im restlichen Teil des Führungsdrahts oder der Seele ist bzw. sehr gering ist bzw. dass dort keine Glasfasern vorhanden sind. Somit enthält die flexible Spitze vorzugsweise nur noch ht-Fasern bzw. ein ht-Faser-Matrix-Komposit, so dass sie plastisch verformbar ist. Dies ist in der klinischen Praxis für die schnelle Anpassung der flexiblen Spitze an die Gefäßstrukturen in einer Zielregion erwünscht. Die flexible Spitze kann vom Arzt selbst, ohne Zuhilfenahme von Wasserdampf und/oder andweitiger Erwärmung, in die gewünschte Form gebracht werden. Somit ist ein derartiger Mikro-Führungsdraht äußerst flexibel einsetzbar und kostengünstig herstellbar, da keine verschiedenen Spitzenformen vorgefertigt werden müssen. Zudem erlaubt die freie plastische Verformbarkeit eine wesentlich bessere Anpassung an die Bedürfnisse des anwendenden Arztes.

Wenn der Anteil an Glasfasern dagegen wesentlich höher ist als der an ht-Fasern, können derartige flexible Spitzen durch Wärmeeinwirkung in eine definierte vorgeformte und nicht einfach zu verändernde Krümmung bzw. mit einem gewünschten Krümmungsradius beaufschlagt werden.

Ein stabförmiger Körper für einen Mikro-Führungsdraht umfasst vorzugsweise im Zentralabschnitt ein oder zwei ht-Faserbündel und mehrere Glasfaserbündel im Peripherabschnitt.

Weiterhin kann der Mikro-Führungsdraht von einem Hüllmaterial umgeben sein, z.B. Polyamid oder Polyurethan. Das Hüllmaterial kann auch z.B. durch einen Schrumpfschlauch (vorzugsweise aus PTFE [Polytetrafluorethylen] oder FEP [Fluoroethylen-Propylen]) ausgebildet werden. Ein Schrumpfschlauch verbessert die Eigenschaften hinsichtlich der Reißfestigkeit des Führungsdrahts gegenüber einem z.B. durch Extrusion aufgebrachten Hüllmaterial.

Durch die Ausbildung der Oberfläche mit einem eine möglichst geringe Reibung aufweisenden Material, z.B. PTFE, werden zudem die mechanischen Eigenschaften des betreffenden medizinischen Instruments weiter verbessert. Z.B. weisen Führungsdrähte aus faserverstärkten Polymeren (FVP) gegenüber Führungsdrähten mit Metallseelen aufgrund der anderen Materialeigenschaften zumeist nicht ganz optimale Torquierungseigenschaften auf. Durch das Vorsehen der minimalen Friktion an z.B. einer Blutgefäßwand oder einer Katheterwand resultierend aus der reibungsarmen Führungsdrahtoberfläche sinken die mechanischen Anforderungen an die Drehsteifigkeit eines solchen FVP-basierten Führungsdrahts.

Hinsichtlich der Mikro-Führungsdrähte ist festzustellen, dass sich fast kein Abstand der MR-Markerpartikel von umgebenden Wasser- oder Fettmolekülen realisieren lässt, wenn der gesamte stabförmige Körper dotiert ist. Das bedeutet, die MR-Markerpartikel sind bei aus dem Stand der Technik bekannten Mikro-Führungsdrähten annähernd direkt benachbart zu umgebenden Wasser- oder Fettmolekülen angeordnet. Dies führt dazu, dass das Artefakt breiter als erwünscht wird, weil nicht, wie bei Standard- und Stiff-Führungsdrähten, die aus mehreren stabförmigen Körpern zusammengesetzt sind (siehe WO 2012/052159 A2), nur der zentrale stabförmige Körper dotiert werden kann, so dass dieser einen größeren Abstand von umgebenden Wasser- oder Fettmolekülen erhält.

Ein erfindungsgemäß ausgebildeter Mikro-Führungsdraht erzeugt somit ein sehr schmales und sehr scharfes Artefakt, da die MR-Markerpartikel im stabförmigen Körper durch die konzentrierte zentrale Anordnung ausreichend weit weg von umgebenden Wasser- oder Fettmolekülen angeordnet sind und somit nur die minimal mögliche Anzahl Voxel geschwärzt wird.

Gemäß einem weiteren Aspekt der vorliegenden Erfindung umfasst ein Führungsdraht zumindest einen zentralen stabförmigen Körper mit Dotierung nur im Zentralabschnitt mit MR-Marker gemäß der vorstehenden Beschreibung und zumindest einen peripheren stabförmigen Körper, wobei der zentrale stabförmige Körper im Zentrum des Führungsdrahtes angeordnet ist und wobei sich der zentrale stabförmige Körper und der periphere stabförmige Körper im Wesentlichen über die gesamte Länge des Führungsdrahtes erstrecken. Der periphere stabförmige Körper weist zumindest ein in ein undotiertes, nicht-ferromagnetisches Matrixmaterial eingebettetes, nicht-metallisches Faserbündel auf. Der zentrale stabförmige Körper und der periphere stabförmige Körper sind in ein nicht-ferromagnetisches Hüllmaterial eingebettet. Zusätzlich kann zur Erhöhung der Reißfestigkeit und für die Erzielung einer optimal glatten Oberfläche ein Schrumpfschlauch aufgebracht werden.

Ein derart erfindungsgemäß ausgebildeter Führungsdraht erzeugt somit ein sehr schmales und sehr scharfes Artefakt, da die MR-Markerpartikel im zentralen stabförmigen Körper durch die konzentrierte zentrale Anordnung optimal konzentiert und optimal weit weg vom umgebenden Wasser angeordnet sind.

Der zentrale stabförmige Körper kann einen Zentralabschnitt und einen Peripherabschnitt aufweisen, wobei der Zentralabschnitt im Zentrum des stabförmigen Körpers angeordnet ist und vom Peripherabschnitt umschlossen ist, wobei sich sowohl der Zentralabschnitt als auch der Peripherabschnitt über die gesamte Länge des stabförmigen Körpers erstrecken, und der Zentralabschnitt zumindest ein Glasfaser-Faserbündel aufweist und der Peripherabschnitt zumindest ein ht-Faserbündel aufweist, wobei beide Faserbündel in ein nicht-ferromagnetisches Matrixmaterial eingebettet sind.

Das Faserbündel des peripheren stabförmigen Körpers kann ein Glasfaserbündel sein.

Der periphere stabförmige Körper kann einen Zentralabschnitt und einen Peripherabschnitt aufweisen, wobei der Zentralabschnitt im Zentrum des stabförmigen Körpers angeordnet ist und vom Peripherabschnitt umschlossen ist, wobei sich sowohl der Zentralabschnitt als auch der Peripherabschnitt über die gesamte Länge des stabförmigen Körpers erstrecken, und der Zentralabschnitt zumindest ein ht-Faserbündel aufweist und der Peripherabschnitt zumindest ein Glasfaserbündel aufweist, wobei beide Faserbündel in ein nicht-ferromagnetisches Matrixmaterial eingebettet sind.

Der Führungsdraht kann einen zentralen stabförmigen Körper und zumindest drei bzw. vier bzw. fünf bzw. sechs bzw. sieben bzw. acht bzw. neun bzw. zehn bzw. zwölf stabförmige Körper aufweisen.

Gemäß einem weiteren Aspekt der vorliegenden Erfindung ist ein Verfahren zur Herstellung eines stabförmigen Körpers gemäß der vorstehenden Beschreibung vorgesehen, wobei ein Zentralabschnitt zumindest eines nicht-metallischen Faserbündels mit einem mit MR-Markerpartikel dotierten, nicht-ferromagnetischen Matrixmaterial ausgebildet wird und auf dem Zentralabschnitt durch Imprägnieren zumindest eines nicht-metallischen Faserbündels mit undotiertem, nicht-ferromagnetischem Matrixmaterial ein Peripherabschnitt ausgebildet wird, so dass dieser den Zentralabschnitt umschließt.

Da in der erfindungsgemäßen Ausführung der stabförmigen Körper die MR-Markerpartikel im Inneren des stabförmigen Körpers angeordnet werden, kommen diese nicht an die Oberfläche des stabförmigen Körpers und beeinträchtigen den Pultrusionsprozess nicht, so wie es der Fall sein kann, wenn die MR-Markerpartikel an der Oberfläche des stabförmigen Körpers liegen und dadurch zu einem ungleichmäßigen Passieren des Ausformwerkzeugs führen können, welches Inhomogenitäten im Pultrudat hervorrufen kann. Je gleichmäßiger der Pultrusionsprozess abläuft, umso höher ist die Produktqualität.

Die geordnete Anordnung der Fasern, insbesondere wenn in einem stabförmigen Körper zwei oder mehr verschiedene Fasertypen enthalten sind, führt zu einer besseren und homogeneren Imprägnierung der Fasern mit dem Matrixmaterial und damit ebenfalls zu einer höheren Produktqualität.

Die Anordnung sämtlicher in einem medizinischen Instrument enthaltenen ht-Fasern in einem einzigen stabförmigen Körper verleiht diesem die bestmögliche Reißfestigkeit und damit eine verbesserte Produktqualität.

Die vorstehend beschriebenen verschiedenen Aspekte der vorliegenden Erfindung sind miteinander kombinierbar.

Der Führungsdraht ist vorzugsweise von einem Schrumpfschlauch, vorzugsweise aus PTFE (Polytetrafluorethylen) oder FEP (Fluoroethylen-Propylen), umgeben. Dieser verbessert dessen Eigenschaften hinsichtlich der Reißfestigkeit und erzeugt eine optimal glatte Oberfläche mit minimaler Reibung, welche die Torquierungseigenschaften des Führungsdrahts weiter verbessert.

Das Hüllmaterial weist in der Regel eine Erweichungstemperatur auf, die unter 300°C bzw. unter 250°C liegt. Der Schrumpfschlauch soll hingegen auf eine Temperatur von über 250°C bzw. von über 300°C, wie z. B. 350°C, erwärmt werden können, um zu schrumpfen und um eng am Führungsdraht anzuliegen. Erwärmt man ein solches medizinisches Instrument, umgebenden Schrumpfschlauch mittels eines Heizgebläses auf eine Temperatur von über 300°C, dann besteht die Gefahr, dass auch das Hüllmaterial des Führungsdrahtes weich wird, und aufgrund des vom Gebläse ausgeübten Luftdruckes das medizinische Instrument platt gedrückt wird. Damit geht die notwendige geometrische Anordnung und/oder die Rundheit des Führungsdrahts verloren.

Zur Vermeidung dieses Problems kann ein von einem noch nicht geschrumpften Schrumpfschlauch umgebener Führungsdraht mittels eines beheizten Rohres erhitzt werden, durch das die Einheit aus Führungsdraht und Schrumpfschlauch hindurchgeführt wird.

Das beheizte Rohr weist typischerweise eine Länge von 1 bis 2 cm auf. Die Länge des beheizten Rohres kann auch bis zu 3 cm bzw. bis zu 5 cm bzw. bis zu 10 cm betragen. Das beheizte Rohr kann auch als kurzer Ring mit einer Länge von nicht weniger als 0,5 cm bzw. einer Länge von nicht weniger als 0,7 cm ausgebildet sein. Der Durchmesser des beheizten Rohres ist derart bemessen, dass darin der Führungsdraht und der Schrumpfschlauch mit geringfügigem Abstand zur inneren Fläche des beheizten Rohres anordbar ist. Dieser Abstand beträgt beispielsweise zumindest 1 mm, wobei er vorzugsweise zumindest 3 mm bzw. zumindest 5 mm beträgt. Das beheizte Rohr weist typischerweise einen Durchmesser von 0,5 bis 5 cm auf. Der Durchmesser beträgt vorzugsweise 0,7 bis 3 cm und insbesondere 0,8 bis 2 cm. Das beheizte Rohr ist vorzugsweise ein gut wärmeleitendes Metallrohr oder rohrähnliches Gehäuse z. B. aus Kupfer, Messing, Bronze, Rotguss, Aluminiumbronze, Zinnbronze, Kupfer-Chrom(I)-Zirkon, Kupfer-Nickel, Kupfer-Aluminium, Neusilber, Ampcoloy®, anderen kupferhaltigen oder kupferbasierten Legierungen oder Stahl. Am Außenumfang oder im Mantel des beheizten Rohres sind Heizelemente angeordnet. Weiterhin weist das beheizte Rohr einen oder mehrer Temperaturfühler auf, um die Temperatur des beheizten Rohres zu messen und exakt einstellen zu können. Da der vom Schrumpfschlauch umgebene Führungsdraht mit Abstand zur inneren Fläche des beheizten Rohres geführt wird, wird die Wärme vom beheizten Rohr auf das medizinische Instrument mittels Strahlung und Konvektion übertragen. Um eine ausreichende Verweilzeit des medizinischen Instrumentes im beheizten Rohr sicherzustellen, sollte die Geschwindigkeit, mit welcher das medizinische Instrument durch das beheizte Rohr geführt wird, nicht größer als 5 m/min, vorzugsweise nicht größer als 2 m/min, noch bzw. nicht größer als 1 m/min und insbesondere nicht größer als 0,75 m/min sein. Um sicherzustellen, dass das Hüllmaterial des Führungsdrahtes beim Schrumpfen des Schrumpfschlauches nicht erweicht, kann die Geschwindigkeit, mit welcher der von einem Schrumpfschlauch umschlossene Führungsdraht bewegt wird, zumindest 0,1 m/min und vorzugsweise zumindest 0,3 m/min und insbesondere zumindest 0,5 m/min betragen. Die tatsächlich notwendige Geschwindigkeit hängt von den Dimensionen und den Materialien, insbesondere dem Hüllmaterial, des Führungsdrahtes und der Dicke des Schrumpfschlauches ab und ist im Einzelfall individuell zu bestimmen. Die vorstehend beschriebene Erhitzung mittels eines beheizten Rohres erlaubt die notwendige Feineinstellung des Wärmeintrags in den Schrumpfschlauch, welcher möglichst hoch sein soll, und in den Führungsdraht, insbesondere das Hüllmaterial, welcher möglichst niedrig sein soll. Durch die freie Kombinierbarkeit von Länge und Durchmesser des beheizten Rohres mit der Prozessgeschwindigkeit und der Prozesstemperatur ohne die Ausübung von Druck auf den erwärmten Führungsdraht wird eine schonende Aufbringung des Schrumpfschlauches auf das Kompositmaterial ermöglicht.

Der Führungsdraht kann ein Mikroführungsdraht sein, der aus einem einzigen stabförmigen Körper ohne Hüllmaterial besteht. Vorzugsweise ist der Führungsdraht ein aus mehreren stabförmigen Körpern bestehender Führungsdraht, die in ein Hüllmaterial eingebettet sind.

Ein solches medizinisches Instrument umfassend einen Führungsdraht und einen Schrumpfschlauch stellt einen eigenständigen Aspekt der vorliegenden Erfindung dar, der unabhängig von den oben erläuterten Aspekten realisiert werden kann. Dieser Aspekt kann selbstverständlich auch in Verbindung mit den oben erläuterten Aspekten der Erfindung ausgeführt werden.

Die Erfindung wird im Folgenden anhand der Zeichnungen näher erläutert. Diese zeigen in:
- Fig. 1: eine perspektivische Ansicht eines stabförmigen Körpers gemäß der vorliegenden Erfindung,
- Fig. 2: den stabförmigen Körper aus Fig. 1 in einer Querschnittsansicht, und
- Fig. 3: einen Führungsdraht gemäß der vorliegenden Erfindung in einer Querschnittsansicht.

Einige der Ausführungsbeispiele der vorliegenden Erfindung sind mit Aramidfaserbündeln ausgebildet. In der nachfolgenden Beschreibung der vorliegenden Erfindung werden die Ausdrücke "Aramidfaser" und "Aramidfilamente" als ein Synonym für ht-Faserbündel verwendet. Aramidfasern sind aufgrund ihrer hohen Zug- bzw. Reißfestigkeit vorgesehen. Daher ist es für den Fachmann klar, dass die Aramidfasern durch andere nicht elektrisch leitfähige Fasern austauschbar sind, die die annähernd gleiche oder eine bessere Zug- bzw. Reißfestigkeit aufweisen.

Der erste Aspekt der vorliegenden Erfindung betrifft einen stabförmigen Körper 1 (im Folgenden auch Stab genannt), der zum einen ein Endprodukt, beispielsweise einen Mikro-Führungsdraht, aber insbesondere ein Zwischenprodukt zur Herstellung von medizinischen Instrumenten, insbesondere Führungsdrähten oder Kathetern bzw. schlauch- oder katheterbasierten Instrumenten oder Seelen für Lead-Sonden darstellt.

Der stabförmige Körper gemäß der vorliegenden Erfindung ist eine Weiterentwicklung der stabförmigen Körper, wie sie in der WO 2007/000148 A2, WO 2009/141165 A2, WO 2012/052159 A2 und WO 2013/041235 A1 beschrieben sind. Daher wird auf die Offenbarung dieser Dokumente vollinhaltlich Bezug genommen und diese Dokumente werden durch Verweisung in die vorliegende Beschreibung einbezogen.

Im Folgenden wird ein erfindungsgemäßer stabförmiger Körper allgemein beschrieben.

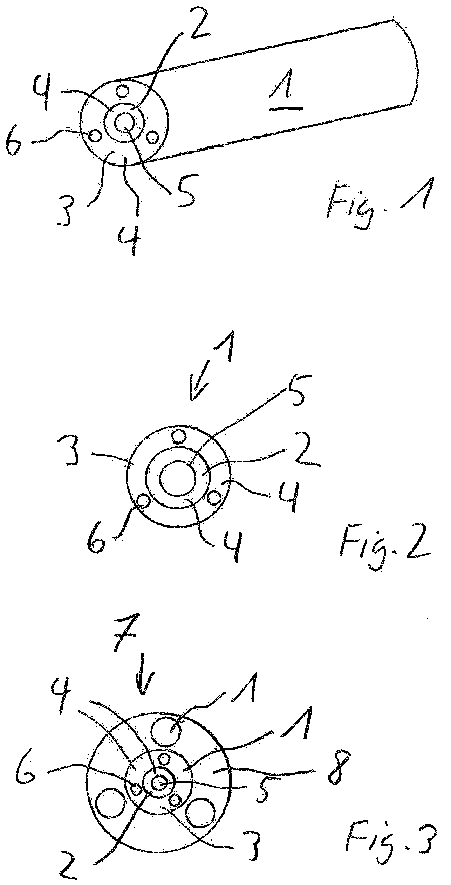

Ein stabförmiger Körper bzw. Stab 1 umfasst einen Zentralabschnitt 2 und einen Peripherabschnitt 3 (Figuren 1 und 2). Der Zentralabschnitt 2 ist im Zentrum des Stabes 1 angeordnet und vom Peripherabschnitt 3 umschlossen.

Sowohl der Zentralabschnitt 2 als auch der Peripherabschnitt 3 erstrecken sich über die gesamte Länge des stabförmigen Körpers.

Der Zentralabschnitt 2 weist zumindest ein in ein nicht-ferromagnetisches Matrixmaterial 4 eingebettetes, nicht-metallisches Faserbündel 5 auf.

Das Faserbündel 5 weist eine Feinheit von 11 Tex auf. Der Zentralabschnitt besitzt einen Durchmesser von etwa 0,1-0,15 mm, insbesondere von maximal 0,15 mm bzw. maximal 0,1 mm.

Das Matrixmaterial im Zentralabschnitt ist mit MR-Markerpartikeln dotiert (nicht dargestellt). Der Peripherabschnitt 3 weist zumindest ein in ein undotiertes, nicht-ferromagnetisches Matrixmaterial 4 eingebettetes, nicht-metallisches Faserbündel 6 auf.

Der stabförmige Körper weist einen Durchmesser kleiner 0,75 mm bzw. kleiner 0,5 mm bzw. kleiner 0,4 mm bzw. kleiner 0,3 mm bzw. kleiner 0,2 mm bzw. kleiner 0,1 mm auf.

Die nicht-metallischen Faserbündel 5, 6 sind elektrisch nicht leitende Fasern bzw. Filamente. Elektrisch leitende Faserbündel würden elektrische Spannung leiten und den stabförmigen Körper 1 durch die während der Magnetresonanztomographie induzierten magnetischen- und RF-Felder aufheizen.

Die stabförmigen Körper 1 können metallische Partikel umfassen, jedoch müssen diese metallischen Partikel derart voneinander beabstandet sein, dass sie keine elektrisch leitenden Bereiche ausbilden.

Das Matrixmaterial umschließt und/oder verklebt die Faserbündel derart, dass diese darin eingebettet sind. Das nicht-ferromagnetische Matrixmaterial 4 des Zentralabschnitts 2 ist mit MR-Markerpartikeln dotiert.

Ein wesentliches Merkmal und ein wesentlicher Vorteil des stabförmigen Körpers 1 ist es, dass verschiedene stabförmige Körper mit einer unterschiedlichen Dotierung mit MR-Markerpartikeln ausgebildet sein können.

In einem Führungsdraht können verschiedene dotierte und/oder undotierte stabförmige Körper integriert sein.

Dies wird im Folgenden anhand der verschiedenen Ausführungsbeispiele der stabförmigen Körper und der Führungsdrähte gemäß der vorliegenden Erfindung näher beschrieben.

Gemäß einem ersten Ausführungsbeispiel des stabförmigen Körpers 1 ist im Zentralabschnitt ein Aramidfaserbündel in das mit MR-Markerpartikeln dotierte Matrixmaterial 4 eingebettet. Das Faserbündel umfasst mehrere, parallel angeordnete, langgestreckte Fasern. Alternativ können die Fasern miteinander verdrillt oder verflochten sein.

In dem den Zentralabschnitt 2 umgebenden Peripherabschnitt 3 sind drei Glasfaserbündel radial umlaufend gleich beabstandet voneinander in das Matrixmaterial 4 eingebettet. Das Matrixmaterial 4 im Peripherabschnitt 3 ist nicht dotiert. Die Glasfaserbündel umfassen vorzugsweise mehrere langgestreckte Fasern, die parallel angeordnet sind.

Alternativ können diese Fasern des Glasfaserbündels auch miteinander verdrillt oder verflochten sein.

Unter "langgestreckt" wird im Rahmen der vorliegenden Erfindung verstanden, dass sich die Faserbündel 5, 6 im Wesentlichen über die gesamte Länge des stabförmigen Körpers 1 erstrecken. Das bedeutet, dass die Länge der Faserbündel 5, 6 zumindest die halbe Länge des stabförmigen Körpers 1 beträgt. Vorzugsweise erstrecken sich die Faserbündel 5, 6 über die ganze Länge des stabförmigen Körpers 1 oder über zumindest 80% bzw. über zumindest 90% bzw. über zumindest 95% der gesamten Länge des stabförmigen Körpers 1.

Derartige langgestreckte Filamente verleihen dem stabförmigen Körper eine hohe Festigkeit in longitudinaler Richtung. Führungsdrähte, die zumindest einen derartigen stabförmigen Körper umfassen, sind dafür ausgebildet, um in ein Blutgefäß, ein Organ (z.B. Herz, Leber, Lunge oder dergleichen) oder das Gehirn einbringbar zu sein. Daher kann eine starke Kraft auf diese Führungsdrähte in longitudinaler Richtung während des Einbringens derselben in das Körperlumen oder beim Herausziehen derselben aus dem Körperlumen aufgebracht werden. Diese Kraft wird durch die stabförmigen Körper 1 aufgenommen.

Zudem müssen die Führungsdrähte eine gewisse Flexibilität aufweisen, um sie entlang Biegungen in dem Körperlumen führen zu können. Dadurch, dass die Faserbündel 5, 6 in longitudinaler Richtung des stabförmigen Körpers angeordnet sind, werden stabförmige Körper ausgebildet, die sowohl eine hohe Festigkeit bzw. Stabilität in longitudinaler Richtung als auch eine ausreichende Flexibilität in lateraler Richtung aufweisen.

Dadurch, dass lediglich das Matrixmaterial des Zentralabschnitts mit MR-Markerpartikeln dotiert ist, wird bei der Magnetresonanztomographie ein besonders schmales und scharfes Artefakt erzeugt.

Eine derart konzentrierte Anordnung einer gegenüber dem Stand der Technik geringeren Menge von MR-Markerpartikeln ist vorteilhaft, da eine geringere Anzahl geschwärzter Voxel den gleichen Schwärzungsgrad ergibt, die Breite des geschwärzten Bereichs aber kleiner ist gegenüber dem Stand der Technik.

Die Artefaktbreite eines erfindungsgemäßen stabförmigen Körpers ist nunmehr fast so schmal wie bei aus dem Stand der Technik bekannten medizinischen Instrumenten, insbesondere Führungsdrähten, die zumindest einen stabförmigen Körper enthalten, da die MR-Markerpartikel bereits in den stabförmigen Körpern durch die erfindungsgemäße konzentrierte zentrale Anordnung ausreichend beabstandet von umgebenden Wasser- oder Fettmolekülen angeordnet sind und nicht erst in z.B. einem Führungsdraht mit einem Hüllmaterial, welches den ausreichenden Abstand zu umgebenden Wasser- oder Fettmolekülen herstellt. Dadurch bewirkt der zusätzliche Abstand zu umgebenden Wasser- oder Fettmolekülen in den Führungsdrähten beispielsweise durch das Vorsehen eines Hüllmaterials nur noch einen geringen, aber keinen wesentlichen zusätzlichen Vorteil in der MRT-Abbildung mehr.

Daher ist es mit einem erfindungsgemäßen stabförmigen Körper mit zentraler Dotierung möglich, ein fast ebenso schmales Artefakt wie mit einem Führungsdraht zu erzeugen.

Die Faserbündel im Peripherabschnitt sind gemäß diesem Ausführungsbeispiel vorzugsweise Glaserfasern.

Es ist auch möglich, dass anstelle der Glasfasern Keramikfasern verwendet werden.

Die Glasfaserbündel im peripheren Abschnitt verleihen dem stabförmigen Körper eine sehr hohe Kompressions- und Biegesteifigkeit.

Die Aramidfaserbündel, die zentral auf einer neutralen Linie angeordnet sind, sind im Gegensatz zu den Glasfaserbündeln, die lediglich elastisch verformbar sind, plastisch verformbar und nehmen dennoch dem stabförmigen Körper fast keine Kompressions- und Biegesteifigkeit weg, sondern verleihen ihm eine bestmögliche Reißfestigkeit.

Auf diese Weise wird ein stark verbesserter stabförmiger Körper bereitgestellt, welcher bestmögliche Eigenschaften hinsichtlich Kompressions- und Biegesteifigkeit sowie Reißfestigkeit aufweist.

Der erfindungsgemäße stabförmige Körper 1 wird durch einen Mikro-Pultrusionsprozess hergestellt. Dabei wird ein Aramidfaserbündel bzw. ein Roving oder ein Yarn zusammen mit einem mit MR-Markerpartikeln dotierten Matrixmaterial 4 zentral angeordnet und der Zentralabschnitt 2 zusammen mit einem oder mehreren Glasfaserbündeln und undotiertem Matrixmaterial pultrudiert.

Gemäß einem zweiten Ausführungsbeispiel des stabförmigen Körpers sind alle Faserbündel als ht-Faserbündel ausgebildet.

Gemäß einem dritten Ausführungsbeispiel der vorliegenden Erfindung ist das Faserbündel im Zentralabschnitt ein Glasfaserbündel und im Peripherabschnitt ein oder mehrere ht-Faserbündel.

Gemäß einem vierten Ausführungsbeispiel des stabförmigen Körpers sind alle Faserbündel als Glasfaserbündel ausgebildet.

Wie bereits vorstehend beschrieben, ist das nicht-ferromagnetische Matrixmaterial im Zentralabschnitt und im Peripherabschnitt das gleiche nicht-ferromagnetische Matrixmaterial mit dem einzigen Unterschied, dass das Matrixmaterial im Zentralabschnitt 2 mit MR-Markerpartikeln dotiert ist und im Peripherabschnitt 3 undotiertes Matrixmaterial vorliegt. Als Matrixmaterial ist vorzugsweise Epoxidharz vorgesehen.

Die MR-Markerpartikel sind passiv-negative MR-Marker, vorzugsweise ausgewählt aus den folgenden Metallen oder Metallverbindungen: Eisen (Fe), Kobalt (Co), Nickel (Ni), Molybdän (Mo), Zirkonium (Zr), Titan (Ti), Mangan (Mn), Rubidium (Rb), Aluminium (AI), Palladium (Pd), Platin (Pt), Chrom (Cr) oder Chromdioxid (CrO₂) oder Eisenoxid (FeO, Fe2O3, Fe3O4).

Die Partikelgröße der passiv-negativen MR-Marker liegt im Bereich von 0 bis 50 µm und insbesondere im Bereich von 0 bis 20 µm, wobei die Größenangabe sich auf den kleinsten Durchmesser bezieht. Die Konzentration der passiven MR-Marker ist so zu wählen, dass das medizinische Instrument bei den gewünschten Sequenzen sichtbar ist, wobei sie das medizinische Instrument in zumindest einer MR-Sequenz gut abbilden, aber die Abbildung des umgebenden Körpergewebes dabei nicht wesentlich überlagern oder stören. Das Gewichtsverhältnis zwischen MR-Markern und dem Matrixmaterial des Zentralabschnitts beträgt etwa 1:5 bis 1:50 und insbesondere 1:10 bis 1:20.

Gemäß einem zweiten Aspekt der vorliegenden Erfindung ist ein Führungsdraht 7 vorgesehen, der vorzugsweise aus einem stabförmigen Körper ausgebildet. Ein solcher Führungsdraht kann ein Mikro-Führungsdraht sein.

Der stabförmige Körper kann gemäß den vorstehend beschriebenen Ausführungen ausgebildet sein, vorzugsweise ist jedoch im Zentralabschnitt 2 ein Aramidfaserbündel angeordnet und im Peripherabschnitt zumindest drei Glasfaserbündel angeordnet. Im Bereich eines als Spitze ausgebildeten distalen Endes des Führungsdrahtes ist der Peripherabschnitt derart radial umlaufend abgeschliffen, dass die Glasfaserbündel zum großen Teil bzw. nahezu vollständig oder ganz entfernt sind.

Auf diese Weise wird eine äußerst flexible und nach den Wünschen des Arztes manuell, d.h. von Hand beliebig verformbare bzw. biegsame distale Spitze bereitgestellt.

Der Mikroführungsdraht ist vorzugsweise von einem PTFE-Schrumpfschlauch (Polytetrafluorethylen) umgeben. Dieser verbessert dessen Eigenschaften hinsichtlich der Reißfestigkeit und erzeugt eine optimal glatte Oberfläche mit minimaler Reibung, welche die Torquierungseigenschaften des Führungsdrahts weiter verbessert.

Gemäß einem weiteren Aspekt der vorliegenden Erfindung ist ein Führungsdraht vorgesehen, der mehrere stabförmige Körper 1 umfasst (Figur 3).

Der Führungsdraht umfasst einen zentralen stabförmigen Körper und zumindest drei periphere stabförmige Körper, wobei der zentrale stabförmige Körper im Zentrum des Führungsdrahtes 7 angeordnet ist.

Die peripheren stabförmigen Körper sind radial in etwa gleich beabstandet um den zentralen stabförmigen Körper herum angeordnet.

Der zentrale stabförmige Körper und der periphere stabförmige Körper erstrecken sich im Wesentlichen über die gesamte Länge des Führungsdrahtes. Die stabförmigen Körper sind in ein nicht-ferromagnetisches Matrixmaterial eingebettet.

Gemäß einem ersten Ausführungsbeispiel dieses Führungsdrahtes weist der zentrale stabförmige Körper einen Durchmesser von zum Beispiel ca. 0,28 mm auf. Als Faserbündel im zentralen stabförmigen Körper sind lediglich Aramidfaserbündel vorgesehen. Diese weisen eine Feinheit von zumindest ca. 6 Tex bzw. zumindest ca. 22 Tex und bevorzugt von zumindest ca. 11 Tex auf, wobei nur das zentrale Aramidfaserbündel im Zentralabschnitt angeordnet mit MR-Markerpartikeln dotiert ist. Die im Peripherabschnitt angeordneten Aramidfaserbündel sind hingegen in nichtdotiertes Matrixmaterial eingebettet. Der Durchmesser des Zentralabschnittes beträgt etwa 0,07 mm bis 0,15 mm. Je größer der Betrag der Feinheit [Tex] ist, desto größer ist auch der Durchmesser des Zentralabschnittes.

Die peripheren stabförmigen Körper weisen einen Durchmesser von zumindest ca. 0,18 mm auf. Diese stabförmigen Körper sind aus Glasfaserbündeln mit einer Feinheit von zumindest ca. 28 oder zumindest ca. 33 Tex ausgebildet, die in Epoxidharz eingebettet sind.

Ein derartiger Führungsdraht umfasst vorzugsweise einen zentralen stabförmigen Körper und drei periphere bzw. sechs periphere bzw. drei bzw. vier bzw. fünf bzw. sechs bzw. sieben bzw. acht bzw. neun bzw. zehn bzw. elf bzw. zwölf periphere stabförmige Körper auf.

Gemäß einem zweiten Ausführungsbeispiel des Führungsdrahtes wird ein zentraler stabförmiger Körper gemäß dem ersten Ausführungsbeispiel des Führungsdrahtes verwendet und die peripheren stabförmigen Körper weisen im Peripherabschnitt Glasfaserbündel mit einer Feinheit von zumindest ca. 28 bzw. zumindest ca. 33 Tex und im Zentralabschnitt Aramidfaserbündel auf, die im Zentralabschnitt der peripheren stabförmigen Körper in mit MR-Markerpartikeln dotiertes Matrixmaterial eingebettet sind.

Alternativ zu den beiden vorstehend beschriebenen Ausführungsbeispielen des Führungsdrahtes kann der zentrale stabförmige Körper im Zentralabschnitt Glasfaserbündel aufweisen, die in dotiertes Matrixmaterial eingebettet sind und wobei im Peripherabschnitt Aramidfaserbündel in undotiertes Matrixmaterial eingebettet sind.

Im Allgemeinen weisen die Glasfaserbündel in den stabförmigen Körpern eine Feinheit von 5-100 Tex und vorzugsweise 20-70 Tex auf. Der Durchmesser der stabförmigen Körper beträgt vorzugsweise zwischen 0,1 mm und 0,4 mm.

Sollten während der medizinischen Intervention eine oder mehrere oder alle Glasfaserbündel eines stabförmigen Körpers brechen, werden die medizinischen Instrumente dennoch durch die Aramidfasern zusammengehalten und können demgemäß sicher entfernt werden.

Die stabförmigen Körper dieses Führungsdrahtes 7 sind in ein Hüllmaterial eingebettet. Dies können zum Beispiel biokompatible Kunststoffe wie Polyamide, PEEK, PEBAX, Polyethylen, Polypropylen, Polyurethan, EVA, PVP, PVC, Silikon, Polymilchsäurepolymere, Mediprene®, Gummimaterialien oder Gedächtniskunststoffe sein.

Der Führungsdraht 7 ist vorzugsweise von einem PTFE-Schrumpfschlauch (Polytetrafluorethylen) umgeben. Dieser verbessert dessen Eigenschaften hinsichtlich der Reißfestigkeit und erzeugt eine optimal glatte Oberfläche mit minimaler Reibung, welche die Torquierungseigenschaften des Führungsdrahts weiter verbessert.

In einem weiteren Ausführungsbeispiel ist das Hüllmaterial aus modifizierten Zusammensetzungen ausgebildet, die vor allem durch mechanisches Mischen erzeugt werden können. Diese chemischen Zusammensetzungen weisen eine oder mehrere freie funktionelle Gruppen, insbesondere Amino- und/oder Carboxyl-Gruppen auf. Diese chemischen Zusammensetzungen umfassen insbesondere Polycarbonsäuren (z.B. Polyacrylsäure, Polyvinylamin, Polyethylenimin, Acrolein-Acrylsäure-Copolymer oder Polyallylamin). Besonders bevorzugt ist eine Zusammensetzung aus einem Hüllmaterial und Polycarbonsäure. Insbesondere wird eine Natriumsalzlösung aus Polycarbonsäure (z.B. POC AS 5060, Evonik Industries, Essen, Deutschland) mit dem Hüllmaterial gemischt, bevorzugt um eine Menge von 5, 10, 20, 30 oder 40 oder mehr als 40 % (w/w) POC im Hüllmaterialzu erhalten. Alle Mengen dazwischen sind auch geeignet und können verwendet werden. Dieses modifizierte Hüllmaterial wird dann z.B. in einem Extrusionsverfahren verwendet, um die stabförmigen Körper zu umschließen und einzubetten oder durch Beschichten der abgeschliffenen flexiblen Spitze eines Führungsdrahts aufgetragen. Die freien Carboxyl-Gruppen an den Oberflächen reagieren vorzugsweise mit Polyvinylamin oder anderen Polyamino-Polymeren, um Amid-Bindungen an der Oberfläche zu ergeben. Verbleibende freie Aminogruppen werden dann vorzugsweise mit kurzkettigen (z.B. C₁-C₆) hydrophilen alpha-omega homobifunktionalen Linkern quervernetzt. Die gleitfähige Oberflächenbeschichtung des medizinischen Instrumentes wird auf diese Weise stabil am medizinischen Instrument befestigt. Diese modifizierten Oberflächen sind geeignet zum Binden von passiv-positiven MR-Markern wie z.B. Gadolinium-Ionen oder Gadolinium-Komplexen oder Cerium- oder Praseodym- oder Neodym- oder Promethium- oder Samarium- oder Europium- oder Terbium- oder Dysprosium- oder Holmium- oder Erbium- oder Thulium- oder Ytterbium- oder Lutetium-Ionen oder - Komplexen.

Im Folgenden werden die Führungsdrähte ausschließlich nach ihren mechanischen Eigenschaften in vorbestimmte Klassen unterteilt. Der Faseranteil bedeutet den gesamten Faseranteil des jeweiligen stabförmigen Körpers und setzt sich aus einem oder mehreren Faserbündeln zusammen und kann aus einem oder mehreren Fasertypen (z.B. ht-Faser und Glasfaser) bestehen. Die Führungsdrähte weisen jeweils einen einzigen erfindungsgemäßen, dotierten stabförmigen Körper auf. Die weiteren stabförmigen Körper sind vorzugsweise nicht dotiert. Der dotierte stabförmige Körper bildet in der Regel den zentralen stabförmigen Körper des Führungsdrahtes, sofern ein zentraler stabförmiger Körper vorhanden ist:

### 1) Steifer oder super-steifer Führungsdraht

Ein steifer oder super-steifer Führungsdraht hat einen Durchmesser von 0,88 bis 0,97 mm (0,035 - 0,038 Inch). Der zentrale stabförmige Körper weist einen Faseranteil von 40-80 Tex oder höher auf, wobei er bis etwa 100 Tex aufweisen kann. Es sind zumindest 5 und insbesondere zumindest 6 oder mehr periphere stabförmige Körper vorgesehen. Der Faseranteil dieser stabförmigen Körper liegt im Bereich von 20 bis 40 Tex. Das Hüllpolymer kann aus einem mittleren oder harten Polymermaterial ausgebildet sein. Je weicher das Hüllpolymer ist, desto steifer sind die stabförmigen Körper. Aufgrund der peripheren Anordnung der peripheren stabförmigen Körper bewirkt eine geringe Erhöhung der Steifigkeit der peripheren stabförmigen Körper eine wesentliche Erhöhung der gesamten Steifigkeit des Führungsdrahtes. Daher kann die gesamte Steifigkeit des Führungsdrahtes einfach durch Verändern der Steifigkeit der peripheren Führungsdrähte an die jeweiligen Bedürfnisse angepasst werden.

### 2) Standardführungsdraht

Der Standardführungsdraht hat typischerweise einen Durchmesser im Bereich von 0,81 bis 0,88 mm (0,032 - 0,035 Inch). Der zentrale stabförmige Körper weist einen Faseranteil von 40 bis 80 Tex auf. Der Standardführungsdraht umfasst zwei bis vier periphere stabförmige Körper. Die peripheren stabförmigen Körper weisen vorzugsweise einen Faseranteil von 20 bis 40 Tex auf. Das Hüllpolymer kann aus einem weichen, mittleren oder harten Polymermaterial ausgebildet sein. Je weicher das Hüllpolymer ist, desto steifer sind die stabförmigen Körper. Aufgrund der peripheren Anordnung der peripheren stabförmigen Körper bewirkt eine geringe Erhöhung der Steifigkeit der peripheren stabförmigen Körper eine wesentliche Erhöhung der gesamten Steifigkeit des Führungsdrahtes. Daher kann die gesamte Steifigkeit des Führungsdrahtes einfach durch Verändern der Steifigkeit der peripheren Führungsdrähte an die jeweiligen Bedürfnisse angepasst werden.

### 3) Standard-Mikroführungsdraht

Ein Standard-Mikroführungsdraht weist einen Durchmesser von etwa 0,30 bis 0,46 mm (0,012 - 0,018 Inch) auf. Der zentrale stabförmige Körper weist einen Faseranteil von vorzugsweise 20 bis 70 Tex und insbesondere 40 bis 60 Tex auf. Der Mikroführungsdraht umfasst vorzugsweise ein bis drei periphere stabförmige Körper.

Der Faseranteil der peripheren stabförmigen Körper beträgt vorzugsweise etwa 10 bis 30 Tex.

Gemäß einer weiteren Ausführungsform des Standard-Mikroführungsdrahtes umfasst dieser lediglich einen einzigen stabförmigen Körper mit einem Faseranteil von 70 bis 130 Tex, und insbesondere von 80 bis 110 Tex. Dieser stabförmige Körper weist passiv-positive MR-Marker, wie z.B. Eisenpartikel, auf. Die Konzentration der passiv-positiven MRI-Marker im Matrixmaterial liegt im Bereich von 1:5 bis 1:30, und vorzugsweise im Bereich von 1:10 bis 1:20. Die Teilchengröße der passiv-positiven MR-Marker-Partikel beträgt vorzugsweise 0 µm bis etwa 20 µm.

Das Hüllpolymer kann aus einem weichen, mittleren oder harten Polymermaterial ausgebildet sein. Je weicher das Hüllpolymer ist, desto steifer sind die stabförmigen Körper. Aufgrund der peripheren Anordnung der peripheren stabförmigen Körper bewirkt eine geringe Erhöhung der Steifigkeit der peripheren stabförmigen Körper eine wesentliche Erhöhung der gesamten Steifigkeit des Führungsdrahtes. Daher kann die gesamte Steifigkeit des Führungsdrahtes einfach durch Verändern der Steifigkeit der peripheren Führungsdrähte an die jeweiligen Bedürfnisse angepasst werden.

### 4) Flexibler Mikroführungsdraht

Der flexible Mikroführungsdraht weist einen Durchmesser von etwa 0,20 bis 0,25 mm (0,008 - 0,010 Inch). Er besitzt nur einen zentralen stabförmigen Körper. Der zentrale stabförmige Körper ist mit einem Faseranteil von vorzugsweise 40 - 80 Tex ausgebildet. Das Hüllpolymer kann aus einem weichen oder mittlerharten Polymermaterial ausgebildet sein.

Im Folgenden wird ein erfindungsgemäßes Verfahren zur Herstellung eines stabförmigen Körpers beschrieben.

Gemäß diesem weiteren Aspekt der vorliegenden Erfindung wird ein Zentralabschnitt zumindest eines nicht-metallischen Faserbündels mit einem mit MR-Markerpartikel dotierten, nicht-ferromagnetischen Matrixmaterial ausgebildet und auf dem Zentralabschnitt durch Imprägnieren zumindest eines nicht-metallischen Faserbündels mit undotiertem, nicht-ferromagnetischem Matrixmaterial ein Peripherabschnitt ausgebildet, so dass dieser den Zentralabschnitt umschließt. Auf diese Weise wird der stabförmige Körper ausgebildet.

Hinsichtlich vorstehend im Einzelnen nicht näher erläuterter Merkmale der Erfindung wird im Übrigen ausdrücklich auf die Patentansprüche und die Zeichnung verwiesen.

### Bezugszeichenliste

1 Stab
2 Zentralabschnitt
3 peripherer Abschnitt
4 nicht-ferromagnetisches Matrixmaterial
5 nicht-metallisches Faserbündel
6 nicht-metallisches Faserbündel
7 Führungsdraht
8 Hüllpolymer

## Patentansprüche

1. Führungsdraht umfassend einen Körper aus Kompositmaterial bestehend aus nicht-metallischen Fasern und einem Matrixmaterial, wobei das Kompositmaterial von einem Schrumpfschlauch umhüllt ist.

2. Führungsdraht nach Anspruch 1,
**dadurch gekennzeichnet,**
**dass** der Körper aus Kompositmaterial aus einem einzelnen stabförmigen Körper (1) aus nicht-metallischen Fasern (6) und einem nicht-ferromagnetischen Matrixmaterial (4) oder aus mehreren stabförmigen Körpern (1) aus nicht-metallischen Fasern (6) und einem Matrixmaterial (4), welche in ein Hüllmaterial eingebettet sind.

3. Führungsdraht nach Anspruch 2,
**dadurch gekennzeichnet,**
**dass** der Führungsdraht mehrere stabförmige Körper aufweist, die in ein Hüllmaterial eingebettet sind, das eine Erweichungstemperatur von unter 300°C aufweist.

4. Führungsdraht nach einem der Ansprüche 1 bis 3,
**dadurch gekennzeichnet,**
**dass** der Schrumpfschlauch aus PTFE (Polytetrafluorethylen) oder FEP (Fluoroethylen-Propylen) ausgebildet ist.

5. Verfahren zum Aufbringen eines Schrumpfschlauches auf einen Führungsdraht, umfassend die folgenden Schritte,
Aufbringen eines Schrumpfschlauches auf den Führungsdraht, und
Erwärmen des Schrumpfschlauches.

6. Verfahren gemäß einem der Ansprüche 5,
**dadurch gekennzeichnet,**
**dass** ein von einem noch nicht geschrumpften Schrumpfschlauch umgebener Führungsdraht mittels eines beheizten Rohres erhitzt wird, durch das die Einheit aus Führungsdraht und Schrumpfschlauch hindurchgeführt wird.

7. Verfahren gemäß Anspruch 6,
**dadurch gekennzeichnet,**
**dass** der vom Schrumpfschlauch umgebene Führungsdraht mit Abstand zu einer inneren Fläche des beheizten Rohres geführt wird.

8. Verfahren gemäß Anspruch 6 oder 7,
**dadurch gekennzeichnet,**
**dass** eine Geschwindigkeit, mit welcher der Führungsdraht durch das beheizte Rohr geführt wird, nicht größer als 5 m/min, vorzugsweise nicht größer als 2 m/min, bzw. nicht größer als 1 m/min und insbesondere nicht größer als 0,75 m/min ist, oder dass die Geschwindigkeit, mit welcher der von einem Schrumpfschlauch umschlossene Führungsdraht bewegt wird, zumindest 0,1 m/min und vorzugsweise zumindest 0,3 m/min und insbesondere zumindest 0,5 m/min beträgt.

9. Führungsdraht nach einem der Ansprüche 1 bis 4,
**dadurch gekennzeichnet,**
**dass** der Führungsdraht gemäß einem Verfahren nach einem der Ansprüche 5 bis 8 hergestellt ist.

10. Vorrichtung zum Aufbringen eines Schrumpfschlauches auf ein medizinisches Instrument oder einen stabförmigen Körper, insbesondere zum Ausführen eines Verfahrens gemäß einem der Ansprüche 1 bis 6, umfassend
eine Heizeinrichtung.

11. Vorrichtung gemäß Anspruch 10,
**dadurch gekennzeichnet,**
**dass** die Heizeinrichtung ein beheiztes Rohr ist, das insbesondere eine Länge von in etwa 1 bis 2 cm oder bis zu 3 cm bzw. bis zu 5 cm bzw. bis zu 10 cm aufweist oder, dass das beheizte Rohr als kurzer Ring mit mi einer Länge von nicht weniger als 0,5 cm bzw. einer Länge von nicht weniger als 0,7 cm ausgebildet ist.

12. Vorrichtung gemäß Anspruch 10,
**dadurch gekennzeichnet,**
**dass** der Durchmesser des beheizten Rohres derart bemessen ist, dass darin der Führungsdraht und der Schrumpfschlauch mit geringfügigem Abstand zur inneren Fläche des beheizten Rohres angeordnet wird, wobei der Abstand zumindest 1 mm, wobei er vorzugsweise zumindest 3 mm bzw. zumindest 5 mm beträgt, und wobei das beheizte Rohr einen Durchmesser von 0,5 bis 5 cm aufweist, wobei der Durchmesser vorzugsweise 0,7 bis 3 cm und insbesondere 0,8 bis 2 cm beträgt.

13. Vorrichtung gemäß einem der Ansprüche 10 bis 12,
**dadurch gekennzeichnet,**
**dass** das beheizte Rohr ein gut wärmeleitendes Metallrohr oder rohrähnliches Gehäuse z. B. aus Kupfer, Messing, Bronze, Rotguss, Aluminiumbronze, Zinnbronze, Kupfer-Chrom(I)-Zirkon, Kupfer-Nickel, Kupfer-Aluminium, Neusilber, Ampcoloy®, anderen kupferhaltigen oder kupferbasierten Legierungen oder Stahl ist.

14. Vorrichtung gemäß einem der Ansprüche 10 bis 13,
**dadurch gekennzeichnet,**
**dass** an einem Außenumfang oder im Mantel des beheizten Rohres Heizelemente angeordnet sind.

15. Vorrichtung gemäß einem der Ansprüche 10 bis 14,
**dadurch gekennzeichnet,**
**dass** das beheizte Rohr einen oder mehrere Temperaturfühler aufweist, um die Temperatur des beheizten Rohres zu messen und exakt einstellen zu können.
